(19) **Europäisches Patentamt**

**European Patent Office**

**Office européen des brevets**

(11) **EP 0 979 643 B1**

(12) **FASCICULE DE BREVET EUROPEEN**

(45) Date de publication et mention
de la délivrance du brevet:
**10.07.2002   Bulletin 2002/28**

(51) Int Cl.7: **A61K 7/02**, A61K 7/021,
A61K 7/027

(21) Numéro de dépôt: **99401999.0**

(22) Date de dépôt: **06.08.1999**

(54) **Composition de maquillage ou de soin sans transfert**

Abriebfeste schminke oder körperpflegemittel

Make-up or personal care composition without rub off

(84) Etats contractants désignés:
**DE ES FR GB IT**

(30) Priorité: **10.08.1998   FR 9810255**

(43) Date de publication de la demande:
**16.02.2000   Bulletin 2000/07**

(60) Demande divisionnaire:
**01117660.9 / 1 159 950**

(73) Titulaire: **L'OREAL**
**75008 Paris (FR)**

(72) Inventeurs:
 • **Arnaud, Pascal**
   **94240 l'Hay les Roses (FR)**
 • **Auguste, Frédéric**
   **94550 Chevilly-Larue (FR)**

(74) Mandataire: **Dossmann, Gérard**
**Bureau D.A. Casalonga-Josse**
**Paul-Heyse-Strasse 33**
**80336 München (DE)**

(56) Documents cités:
**EP-A- 0 709 083**          **EP-A- 0 847 752**
**EP-A- 0 850 644**          **WO-A-96/15761**
**WO-A-96/40044**          **FR-A- 2 772 601**

 • **PATENT ABSTRACTS OF JAPAN vol. 10, no. 131
   (C-346) & JP 60 255714 A (POLA KASEI KOGYO
   KK)**
 • **HANSEN C.M.: 'The Three Dimensional
   Solubility Parameter' JOURNAL OF PAINT
   TECHNOLOGY vol. 39, no. 505, 1967, pages 104
   - 117**
 • **HANSEN C.M., SKAARUP K.: 'III. Independent
   Calculation of the Parameter Components'
   JOURNAL OF PAINT TECHNOLOGY vol. 39, no.
   511, Août 1967, pages 511 - 514**
 • **VAN KREVELEN D.W.: 'Properties of Polymers',
   1990, ELSEVIER, AMSTERDAM**
 • **BRANDRUP J., IMMERGUT E.H., GRULKE E.A.:
   'Polymer Handbook', 1999, JOHN WILEY &
   SONS, INC., NEW YORK**

## EP 0 979 643 B1

**Description**

La présente invention concerne une composition de maquillage ou de soin de la peau, des fibres kératiniques (cils, sourcils ou cheveux), ou des lèvres, sans transfert renfermant l'association d'une huile hydrocarbonée volatile et d'une cire synthétique fonctionnalisée choisie parmi les alcools gras linéaires en $C_{20-60}$, l'utilisation de cette association pour conférer des propriétés non-transfert à des compositions de maquillage ou de soin de la peau, des fibres kératiniques ou des lèvres, ainsi qu'un procédé de fabrication de telles compositions de maquillageou de soin sans transfert.

La mise au point de produits de maquillage ou de soin dits "sans transfert" fait actuellement l'objet de nombreuses recherches cosmétologiques. Ces produits, par exemple des fonds de teint ou des rouges à lèvres, des fards à paupières ou à joues, se distinguent par le fait qu'une fois appliqués sur la peau ou les lèvres, ils ne se déposent pas de façon notable sur les surfaces avec lesquelles ils viennent en contact (verre, tasse, cigarette, vêtement par exemple).

Une première approche pour empêcher le transfert des produits cosmétiques appliqués a consisté à les recouvrir d'une couche de produits réputés pour leur propriétés anti-adhésives, tels que des produits fluorés ou siliconés. Les formulations de ce type présentent cependant l'inconvénient d'une mauvaise cosméticité. Par exemple, le film de rouge à lèvres devient huileux et susceptible de migrer sur la peau contiguë aux lèvres.

Une autre possibilité pour obtenir des produits "sans transfert" consiste à utiliser des polymères ou résines siliconées en association avec des matières premières volatiles qui laissent, après évaporation de ces dernières, un film inerte résistant au transfert sur d'autres surfaces. Les matières premières volatiles utilisées sont par exemple des silicones cycliques de très faible viscosité (inférieure à 3 centistokes) ou encore des isoparaffines.

Pour que ces produits de maquillage se présentent sous forme de solide, il est nécessaire d'y adjoindre des composés de durcissement tels que des cires. Se posent alors des problèmes de stabilité mécanique et/ou de compatibilité entre les cires et les produits volatils. On constate en effet, pour une faible teneur en cires, une dureté insuffisante du stick qui peut être à l'origine de problèmes de stabilité ou d'utilisation. La simple augmentation de la proportion de cires durcissantes ne permet pas de résoudre ces problèmes car elle se traduit généralement par une dégradation des propriétés cosmétiques du produit qui devient inconfortable à porter.

L'objectif de la présente invention a été de trouver des cires permettant un durcissement suffisant, c'est-à-dire ayant un pouvoir de gélification maximal, de manière à limiter la proportion de ces cires dans les produits cosmétiques solides sans transfert et d'éviter les problèmes d'incompatibilité.

Le problème du transfert existe aussi pour les produits de soin ou de traitement de la peau, contenant des actifs, colorés ou non.

La demanderesse a trouvé de manière inattendue qu'une catégorie particulière de cires, à savoir, les alcools gras linéaires en $C_{20-60}$, permettaient de résoudre les problèmes précédemment évoqués.

L'utilisation de telles cires pour la gélification de véhicules siliconés liquides volatiles tels que les cyclométhicones en vue de la préparation de déodorants-crèmes est décrite dans le document WO 97/17942, mais ce document ne concerne nullement l'obtention de produit solide sans transfert à base d'isoparaffines.

La présente invention a donc pour objet une composition cosmétique de maquillage ou de soin de la peau, des lèvres ou des fibres kératiniques sans transfert comprenant au moins une huile hydrocarbonée volatile et au moins une cire synthétique fonctionnalisée choisie parmi les alcools gras linéaires en $C_{20-60}$.

L'invention a également pour objet l'utilisation des cires synthétiques choisies parmi les alcools gras linéaires en $C_{20-60}$ pour le durcissement de compositions de maquillage ou de soin de la peau, des lèvres et des fibres kératiniques renfermant des huiles hydrocarbonées volatiles, dans le but d'obtenir des compositions cosmétiques solides "sans transfert".

Encore un autre objet de la présente invention est un procédé de préparation de compositions de maquillage ou de soin solides "sans transfert" associant des huiles hydrocarbonées volatiles et des cires synthétiques choisies parmi les alcools gras linéaires en $C_{20-60}$.

La présente invention a aussi pour objet un procédé pour limiter le transfert d'une composition de maquillage ou de soin de la peau, des lèvres ou des fibres kératiniques sur une surface avec laquelle, la peau, les lèvres ou les fibres kératiniques sont mise en contact, consistant à introduire dans la composition l'association des huiles hydrocarbonées et de cires synthétiques choisies parmi les alcools gras linéaires en $C_{20-60}$.

Les compositions cosmétiques solides "sans transfert" de la présente invention comprennent donc, dans une phase grasse,

- au moins une huile hydrocarbonée volatile et
- au moins une cire synthétique fonctionnalisée choisie parmi les alcools gras linéaires en $C_{20-60}$, cette cire ayant un point de fusion compris entre 75 °C et 120 °C et des paramètres de solubilité de Hansen ($\delta_d$, $\delta_p$, et $\delta_h$) tels que :

$$15,50 \leq \delta_d \leq 18,50 \; J^{1/2}/cm^{3/2}$$

$$et \; 4,50 \leq \delta_a \leq 7,50 \; J^{1/2}/cm^{3/2}$$

avec

$$\delta_a = (\delta_p{}^2 + \delta_h{}^2)^{1/2}.$$

On entend par huile volatile dans la présente invention, une huile susceptible de s'évaporer à température ambiante d'un support sur lequel elle a été appliquée, autrement dit une huile ayant une tension de vapeur mesurable à température ambiante, et par huile hydrocarbonée volatile une huile dont le squelette ne contient que des hydrogènes et des carbones.

Les huiles hydrocarbonées volatiles préférées convenant dans la présente invention sont en particulier les isoparaffines, à savoir des alcanes ramifiés, comportant de 8 à 16 atomes de carbone. On peut bien entendu également utiliser des mélanges de telles isoparaffines.

L'huile volatile hydrocarbonée utilisée de préférence pour la présente invention est l'isododécane, c'est-à-dire le 2,2,4,4,6-pentaméthylheptane.

On peut citer à titre d'exemple d'isododécane disponible sur le marché le produit commercialisé sous la dénomination PERMETHYL® 99A par la société PRESPERSE Inc.

Les cires synthétiques fonctionnalisées utilisées pour la gélification et le durcissement des compositions de maquillage de la présente invention présentent l'avantage d'être des produits de composition constante et avoir une teneur en alcool gras constante, à l'inverse des cires d'origine naturelle. Elles sont caractérisées, de plus, par leur température de fusion et par leur paramètres de solubilité dans l'espace tridimensionnel de solubilité selon Hansen (*The three-dimensional solubility parameters*, J. Paint Technol., <u>39</u>, page 105 (1967)).

Selon Hansen, le changement d'énergie interne lors de la vaporisation est considéré comme étant la somme de trois contributions, la première due aux liaisons hydrogène (indice h), la seconde aux interactions dipolaires (indice p) et la troisième aux forces de dispersion (indice d).

Le paramètre de solubilité $\delta$ (défini comme la racine carrée de la variation d'énergie interne par unité de volume de l'espèce chimique lors de sa vaporisation et exprimé en $(J/cm^3)^{1/2}$ est défini comme un vecteur de coordonnées ($\delta_h$, $\delta_p$ et $\delta_d$), l'amplitude du vecteur étant donnée par

$$\delta^2 = \delta_h{}^2 + \delta_p{}^2 + \delta_d{}^2$$

Le paramètre $\delta_a$ utilisé dans la présente invention comme critère de sélection des cires appropriées est défini par l'équation suivante :

$$\delta_a = (\delta_p{}^2 + \delta_h{}^2)^{1/2}$$

Le pouvoir gélifiant des cires synthétiques fonctionnalisées de la présente invention est lié aux phénomènes de compatilibité/incompatibilité de celles-ci avec la phase grasse, notamment avec les isoalcanes.

Des cires ayant un paramètre $\delta_a$ inférieur à 4,5 présentent une trop grande compatibilité avec les isoalcanes et ont par conséquent un pouvoir gélifiant et structurant insuffisant. D'autre part, un paramètre $\delta_a$ supérieur à 7,5 est le signe d'une trop forte incompatibilité entre la cire et les isoalcanes qui se traduit par des phénomènes de démixion, d'inhomogénéité et éventuellement par un manque de stabilité mécanique.

Selon la présente invention, les cires remplissant ces critères de sélection sont les alcools gras linéaires à très longue chaîne correspondant à la formule chimique suivante

$$(1) \qquad CH_3\text{-}(CH_2)_n\text{-}CH_2OH$$

dans laquelle n est un entier variant de 18 à 58.

De tels alcools gras en $C_{20\text{-}60}$ sont disponibles dans le commerce par exemple auprès de la société NEW PHASE TECHNOLOGIES sous les dénominations PERFORMACOL® 350, PERFORMACOL® 425, PERFORMACOL® 550 et PERFORMACOL® 700, ou par la société PETROLITE sous les dénominations UNILIN® 350 Alcohol, UNILIN® 425 Alcohol, UNILIN® 550 Alcohol et UNILIN® 700 Alcohol. Il s'agit de mélanges d'alcools linéaires à très longue chaîne obtenus par un procédé de polymérisation permettant d'obtenir des polymères à très faible indice de polydispersité ($M_p/M_n \approx 1,1$). Leur masse molaire moyenne en poids est comprise entre environ 350 et 1000.

Dans les compositions de maquillage sans transfert de la présente invention, l'huile hydrocarbonée volatile représente généralement de 5 à 90 %, de préférence de 5 à 80 % et mieux de 10 à 60% en poids de la composition cosmétique totale. Ces huiles hydrocarbonées peuvent représenter 100% de la phase volatile présente dans la composition.

La ou les cires synthétiques fonctionnalisées représentent généralement de 5 % à 30 %, de préférence de 8 % à 20 % en poids de la composition totale.

La phase grasse des compositions cosmétiques de la présente invention peut contenir, outre les huiles hydrocarbonées volatiles, en particulier les isoparaffines en $C_{8-16}$, et les cires synthétiques fonctionnalisées choisies parmi les alcools gras linéaires en $C_{20-60}$, une ou plusieurs cires d'origine animale, végétale ou synthétique différentes des alcools gras en $C_{20-60}$ décrits ci-dessus, ayant de préférence un point de fusion supérieur à 30°C et idéalement supérieur à 45°C. Ces cires sont choisies, entre autres, parmi la lanoline éventuellement hydrogénée, hydroxylée ou acétylée, la cire d'abeille, le spermacéti, les alcools de lanoline, les acides gras de lanoline et l'alcool de lanoline acétylée, la cire de Carnauba, de Candellila, de kapok, d'Ouricury, de riz, de jojoba hydrogénée, d'Alfa ou du Japon, les cires de fibres de liège ou de canne à sucre, le beurre de cacao, les cires de paraffine, de lignite, de pétrolatum, de vaseline ou les cires microcristallines, la cérésine, l'ozokérite, la cire de montan, les cires de polyéthylène, les cires obtenues par synthèse de Fischer-Tropsch, les esters linéaires résultant de la réaction d'un acide carboxylique saturé en $C_{10-40}$ et d'un alcool saturé en $C_{10-40}$, l'alcool cétylique, l'alcool stéarylique, les lanolates ou stéarates de calcium, les huiles de ricin, de palme, de coco, de tournesol ou de coprah hydrogénées.

Elles peuvent contenir également un ou plusieurs solvants volatils supplémentaires, différents des huiles hydrocarbonées volatiles de la présente invention. On peut citer à titre d'exemple de ces solvants volatils supplémentaires les huiles de silicones cycliques et volatiles telles que les cyclométhicones($D_4$, $D_5$, $D_6$), les silicones linéaires volatiles telles que le décaméthyltétrasiloxane ($L_4$), l'octylheptaméthyltrisiloxane, l'octaméthyltrisiloxane ($L_3$), l'hexylheptaméthyltrisiloxane, et les huiles volatiles fluorées telles que le nonafluorométhyoxybutane ou le perfluorométhylcyclopentane.

Ces solvants volatils représentent de préférence de 0 à 50% en poids de la phase volatile.

Les compositions de la présente invention peuvent contenir également des huiles siliconées et/ou hydrocarbonées et/ou fluorées non volatiles, des gommes de silicone et des cires de silicone.

Les huiles de silicone non volatiles utilisables clans la composition selon l'invention peuvent être des huiles de faible viscosité telles que les polysiloxanes linéaires, dont le degré de polymérisation est de préférence de 6 à 2000 environ. On peut citer, par exemple: les polydiméthylsiloxanes (PDMS) de viscosité supérieure à 10 mPa.s, les phényldiméthicones, les phényltriméthicones, les polyphénylméthylsiloxanes et leurs mélanges.

Les gommes de silicone utilisables dans le produit de l'invention peuvent être des polysiloxanes de masse moléculaire élevée, de l'ordre de 200 000 a 1 000 000 et ayant une viscosité supérieure à 500 000 mPa.s. Elles peuvent être utilisées seules ou en mélange avec un solvant tel qu'une huile polydiméthylsiloxane ou polyphénylsiloxane.

Les gommes peuvent être présentes a raison de 0 a 2% en poids de matière active dans le produit coule final, de préférence a raison de 0,1 a 1%.

Les cires de silicone utilisable dans la composition selon l'invention peuvent être des polysiloxanes linéaires substitués. On peut citer, par exemple, les cires de silicone polyéther, les alkyl ou alkoxydiméthicones ayant de 16 a 45 atomes de carbone. Ces cires de silicone peuvent être présentes à raison de 0 à 18% en poids dans la composition finale, de préférence à raison de 2 a 15%.

Les huiles hydrocarbonées utilisables dans la composition selon l'invention peuvent être des huiles d'origine végétale, animale, minérale ou synthétique.

Comme huiles hydrocarbonées non volatiles utilisables dans l'invention, on peut citer notamment:

- les huiles hydrocarbonées d'origine animale telles que le perhydrosqualène;
- les huiles hydrocarbonées végétales telles que les triglycérides liquides d'acides gras de 4 à 10 atomes de carbone comme les triglycerides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité;
- les hydrocarbures linéaires ou ramifiés, d'origine minérale ou synthétique tels que la vaseline, les polydécènes, le polyisobutène hydrogéné tel que le parléam;
- les esters et les éthers de synthèse comme les huiles de formule $R_1COOR_2$ dans laquelle $R_1$ représente le reste

d'un acide gras supérieur comportant de 6 à 29 atomes de carbone et $R_2$ représente une chaîne hydrocarbonée contenant de 3 à 30 atomes de carbone, telles que l'huile de Purcellin, le myristate d'isopropyle, le palmitate d'éthyl-2 hexyle, le stéarate d'octyl-2 dodécyle, l'érucate d'octyl-2 dodécyle, l'isostéarate d'isostéaryle, le propionate d'arachidyle, le benzoate d'octyl-2 dodecyle ; les esters hydroxylés comme l'isostéaryl lactate, l'octylhydroxystéarate, l'hydroxystéarate d'octyldodécyle, le diisostéarylmalate, le citrate de triisocétyle ; les esters de polyol comme le dioctanoate de propylène glycol, le diheptanoate de néopentylglycol, le diisononanoate de diéthylèneglycol et les esters du pentaérythritol ;

- les alcools gras liquides à température ambiante à chaîne carbonée ramifiée et/ou insaturée ayant de 12 à 24 atomes de carbonne comme l'octyldodécanol, le 2-hexyldécanol, le 2-butyloctanol, le 2-undécylantadécanol, l'alcool isostéarylique oléique ; leurs mélanges.

Ces huiles non volatiles sont présentes de préférence en une quantité allant de 5 à 60 % en poids de la composition totale.

Les compositions cosmétiques sans transfert peuvent contenir bien entendu également les principes actifs qui leur confèrent leur propriétés cosmétiques caractéristiques et des adjuvants cosmétiques. Il s'agit par exemple de substances telles que les filtres solaires, les agents anti-radicaux libres, les agents hydratants, les vitamines, les protéines, les céramides, les agents régulateurs de pH, les anti-oxydants, les agents conservateurs, les charges, les pigments et colorants, les émollients, les agents anti-mousse, les parfums, les agents tensio-actifs et les plastifiants.

Bien entendu, l'homme de métier veillera à choisir ce ou ces éventuels composés complémentaires et leur quantité de manière à ce que les propriétés avantageuses attachées intrinsèquement à la composition cosmétique de l'invention ne soient pas, ou substantiellement pas, altérées par la ou les adjonctions envisagées.

La composition de l'invention contient avantageusement une phase particulaire généralement présente à raison de 0,05 a 35 % du poids total de la composition, de préférence de 2 à 25 %, et qui peut comprendre des pigments et/ou des nacres et/ou des charges habituellement utilisés dans les compositions cosmétiques. Cette charge peut conduire à une composition colorée, blanche ou incolore.

Par pigments, il faut comprendre des particules blanches ou colorées, minérales ou organiques, insolubles dans la phase grasse liquide, destinées à colorer et/ou opacifier le produit coulé. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 25 % du poids de composition finale, et de préférence a raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium.

Les nacres peuvent être présentes dans la composition à raison de 0 à 20 % du poids total du produit coulé, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré

Les charges peuvent être présentes à raison de 0 à 35 % du poids total de la composition, de préférence 2 a 15 %. On peut notamment citer le talc, le mica, la silice, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

La composition peut également comprendre un tensio-actif, par exemple un tensio-actif usuel anionique ou non ionique. Le tensio-actif est de préférence présent, à raison de 0,5 à 8% en poids de la composition.

Elle peut également comprendre des colorants liposolubles et/ou des colorants hydrosolubles.

Les compositions cosmétiques de l'invention peuvent se présenter sous une forme solide, pâteuse, ne s'écoulant pas sous son propre poids. Il peut s'agir d'émulsions ou de compositions anhydres.

La forme mettant le mieux à profit les propriétés de durcissement de la catégorie de cires utilisée dans la présente invention est bien entendu la forme stick ou coulée en coupelle. Elle constitue par conséquent un mode de réalisation préféré de la présente invention.

Les procédés de fabrication des produits de maquillage ou de soin selon l'invention ne diffèrent en rien des procédés classiquement utilisés en cosmétique et sont parfaitement connus de l'homme de l'art.

Un produit de maquillage solide coulé tel qu'un rouge à lèvres, un fond de teint solide, un produit anti-cernes ou encore un produit de coloration ou de protection de la peau ou un mascara "pain" ou une poudre compacte, sont fabriqués par exemple par

- fusion et mélange des composants non volatils de la composition,
- addition, à une température plus basse, de la phase volatile,

- coulage du mélange ainsi obtenu dans un moule de forme appropriée, et
- refroidissement jusqu'à température ambiante.

L'invention est illustrée plus en détail dans les exemples suivants.

**Exemple 1**

Evaluation de la dureté d'une composition renfermant l'association isododécane + cire

On prépare différents sticks à partir d'une composition type constituée de 85 % en poids d'isododécane (PERMETHYL® 99A de la société PRESPERSE) et de 15 % d'une cire dont la nature chimique varie d'un stick à l'autre. Les sticks sont fabriqués par fusion et mélange de ces deux composants, coulage dans un moule cylindrique approprié et refroidissement. La dureté des sticks obtenus est mesurée à 20°C au moyen d'un dynamomètre DFGHS 2 de la société INDELCO-CHATILLON se déplaçant à une vitesse de 100 mm/minute. Elle est exprimée comme la force de cisaillement (exprimée en grammes) nécessaire pour couper un stick de 12,7 mm de diamètre dans ces conditions.
Le Tableau 1 ci-dessous présente les différentes cires utilisées, leur température de fusion, les paramètres de solubilité $\delta_d$ et $\delta_a$ ainsi que les résultats des essais de dureté obtenus.

Tableau 1

| cire | $T_{fusion}$ (°C) | $\delta_d$ ($J^{1/2}$ cm$^{-3/2}$) | $\delta_a$ ($J^{1/2}$ cm$^{-3/2}$) | force de cisaillement (g) |
|---|---|---|---|---|
| cire de polyéthylène PERFORMALENE 500 (New Phase Technologies) | 86 | 16,29 | 0 | 6 |
| cire de polyéthylène PERFORMALENE655 (New Phase Technologies) | 102 | 16,35 | 0 | 6 |
| stéarate d'octanosyle KESTER WAX82H (Koster Keunen) | 82 | 16,52 | 3,07 | 4 |
| alcool gras linéaire PERFORMACOL700 (New Phase Technologies) | 105 | 16,58 | 4,62 | 73 |
| alcool gras linéaire PERFORMACOL 550 (New Phase Technologies) | 99 | 16,54 | 4,73 | 75 |
| alcool gras linéaire PERFORMACOL 425 (New Phase Technologies) | 91 | 16,51 | 5,55 | 28 |
| hydroxystéarate d'hydroxy octacosanyle ELFACOS C26 (Akzo Nobel) | 80 | 16,75 | 7,9 | 1 |
| huile de ricin hydrogénée CUTINA HR (Henkel) | 86 | 16,96 | 9,01 | non compatible |

Ces résultats des essais de dureté montrent que seules les cires selon la présente invention, à savoir les alcools gras linéaires en C$_{20-60}$ (ayant un paramètre $\delta_a$ compris entre 4,5 et 7,5 $J^{1/2}$/cm$^{3/2}$), permettent d'obtenir des sticks d'une dureté suffisante. Les autres cires, bien qu'ayant une température de fusion et un paramètre $\delta_d$ situés à l'intérieur des intervalles définis dans la présente invention, conduisent à des sticks trop mous ou présentant des problèmes d'incompatibilité.

**Exemple 2**

On a préparé de la manière indiquée dans l'Exemple 1 différents sticks à partir de la composition type suivante :

| cire (voir Tableau 2) | 16 g |
| isododécane (PERMETHYL® 99A, Presperse) | 38,8 g |
| ozokérite (OZOKERITE WAX SP 1020, Strahl Pitsch) | 6,4 g |
| silicone volatile (DC 200 fluide 1,5 cSt, Dow Corning) | 38,8 g |
| | 100 g |

La dureté des sticks est mesurée à 20°C au moyen d'un analyseur de texture TA-XT2 commercialisé par la société RHEO.

La dureté est assimilée à la force maximale (en Newton) mesurée lors de la pénétration d'un cylindre en inox de 5 mm de diamètre sur une profondeur de 0,3 mm et à une vitesse de 0,1 mm/seconde à l'intérieur d'un stick à tester.

Les résultats obtenus sont rassemblés dans le Tableau 2 ci-après.

Tableau 2

| cire | $T_f$ (°C) | $\delta_d$ (J$^{1/2}$ cm$^{-3/2}$) | $\delta_a$ (J$^{1/2}$ cm$^{-3/2}$) | F max (N) |
|---|---|---|---|---|
| cire de polyéthylène PERFORMALENE® 500 (New Phase Technologies) | 86 | 16,29 | 0 | 0,17 |
| cire de polyéthylène A-C 1702 (Allied Chemical) | 92 | 16,3 | 0 | non mesurable trop mou < 0,17 |
| alcool gras linéaire PERFORMACOL® 550 (New Phase Technologies) | 99 | 16,54 | 4,73 | 3,23 |

Ces résultats montrent que les cires synthétiques fonctionnalisées selon la présente invention permettent de durcir de manière satisfaisante une composition complexe susceptible de constituer la base d'un produit de maquillage sans transfert, alors que des cires non conformes à l'invention car ayant un paramètre de solubilité $\delta_a$ non compris dans l'intervalle défini précédemment, aboutissent à des compositions d'une tenue mécanique insatisfaisante.

**Exemple 3**

Rouges à lèvres

On prépare les rouges à lèvres sans transfert A, B, C en stick suivants :

| | A | B comparatif | C comparatif |
|---|---|---|---|
| alcool gras linéaire PERFORMACOL® 550 (New Phase Technologies) | 10,00 | - | - |
| cire de polyéthylène PERFORMALENE® 500 (New Phase Technologies) | | 10,00 | 23,00 |
| isododécane | 40,00 | 40,00 | 33,61 |
| cycopentapolysiloxane | 10,00 | 10,00 | 8,39 |
| polyisobutène hydrogéné | 31,34 | 31,34 | 26,34 |
| DC Red n° 7 Calcium lake | 2,90 | 2,90 | 2,90 |
| oxyde de titane | 1,80 | 1,80 | 1,80 |
| FD et C yellow n°6 Aluminium lake | 3,30 | 3,30 | 3,30 |
| oxyde de fer noir | 0,06 | 0,06 | 0,06 |
| DC Red n° 21 Aluminium lake | 0,60 | 0,60 | 0,60 |
| | 100 % en poids | 100 % en poids | 100 % en poids |
| force de cisaillement (en grammes) | 70 | 8 | 75 |

La comparaison des compositions A et B montre que, pour une teneur en cire donnée, seul l'alcool gras PERFORMACOL® 550 conforme à l'invention donne un rouge à lèvres ayant une dureté satisfaisante.

Pour atteindre une dureté comparable avec une cire non conforme à l'invention, il est nécessaire d'augmenter considérablement la teneur en cire (composition C). L'augmentation de la teneur en cire se traduit cependant par une détérioration des propriétés cosmétiques du stick de rouge à lèvres. En effet, la composition A est considérablement plus glissante, moins sèche et plus homogène que la composition C.

**Revendications**

1.   Composition de maquillage ou de soin sans transfert, **caractérisée par le fait qu'**elle comprend, dans un milieu physiologiquement acceptable l'association,

   -   d'au moins une huile hydrocarbonée volatile constituée uniquement d'atomes de carbone et d'atomes d'hydrogène, et
   -   d'au moins une cire synthétique fonctionnalisée choisie parmi les alcools gras linéaires en $C_{20-60}$.

2.   Composition sans transfert selon la revendication 1, **caractérisée en ce que** l'huile hydrocarbonée volatile est une isoparaffine en $C_{8-16}$ ou un mélange d'isoparaffines en $C_{8-16}$.

3.   Composition sans transfert selon la revendication 2, **caractérisée en ce que** l'huile hydrocarbonée volatile est l'isododécane (2,2,4,4,6-pentaméthylheptane).

4.   Composition sans transfert selon l'une quelconque des revendications précédentes, **caractérisée en ce que** l'huile hydrocarbonée volatile représente de 5 à 90 %, de préférence de 5 à 80 % et mieux de 10 à 60% en poids de la composition totale.

**5.** Composition sans transfert selon l'une quelconque des revendications précédentes, **caractérisée en ce que** la ou les cires synthétiques fonctionnalisées représentent de 5 à 30 %, de préférence de 8 à 20 % en poids de la composition totale.

**6.** Composition sans transfert selon l'une quelconque des revendications précédentes, **caractérisée en ce qu'**elle comprend en outre une ou plusieurs cires d'origine animale, végétale ou synthétique différentes des cires synthétiques fonctionalisées.

**7.** Composition sans transfert selon l'une quelconque des revendications précédentes **caractérisée en ce qu'**elle comprend en outre un ou plusieurs solvants volatils différents des huiles hydrocarbonées volatiles.

**8.** Composition sans transfert selon l'une quelconque des revendications précédentes, **caractérisée en ce qu'**elle comprend en outre une gomme de silicone, de préférence en une quantité au plus égale à 2 % en poids.

**9.** Composition sans transfert selon l'une quelconque des revendications précédentes, **caractérisée en ce qu'**elle comprend en outre au moins une huile non volatile d'origine végétale, animale, minérale ou synthétique, de préférence en une quantité allant de 5 à 60 % en poids de la composition totale.

**10.** Composition sans transfert selon l'une quelconque des revendications précédentes, **caractérisée en ce qu'**elle comprend en outre un ou plusieurs adjuvants ou principes actifs cosmétiques choisis parmi les filtres solaires, les agents anti-radicaux libres, les agents hydratants, les vitamines, les protéines, les céramides, les agents régulateurs de pH, les anti-oxydants, les agents conservateurs, les charges, les pigments et colorants, les émollients, les agents anti-mousse, les parfums, les agents tensioactifs et les plastifiants.

**11.** Composition sans transfert selon l'une quelconque des revendications précédentes, **caractérisée en ce qu'**elle se présente sous la forme d'un produit de maquillage ou de soin solide ou pâteux, anhydre ou en émulsion.

**12.** Composition sans transfert selon la revendication 11, **caractérisée en ce que** le produit de maquillage est un rouge à lèvres, un fond de teint, un fard à paupières ou à joues ou un mascara.

**13.** Composition de maquillage sans transfert selon la revendication 11, **caractérisée en ce que** le produit de maquillage solide est un stick de rouge à lèvres, un fond de teint ou une poudre compacte.

**14.** Utilisation de l'association d'au moins une huile hydrocarbonée volatile constituée uniquement d'atomes de carbone et d'atomes d'hydrogène et d'au moins une cire synthétique fonctionnalisée : choisie parmi les alcools gras linéaires en $C_{20-60}$ pour la fabrication d'un produit cosmétique de maquillage ou de soin sans transfert.

**15.** Procédé pour limiter le transfert d'une composition de soin ou de maquillage de la peau des lèvres, ou des fibres kératiniques sur une surface avec laquelle, la peau, les lèvres ou les fibres sont mises en contact consistant à introduire dans la composition l'association telle que définie dans les revendications 1 à 5.

**Patentansprüche**

**1.** Zusammensetzung zum Schminken oder zur Pflege ohne Transfer, **dadurch gekennzeichnet, dass** sie in einem physiologisch akzeptablen Medium die folgende Kombination enthält:

- mindestens ein flüchtiges Kohlenwasserstofföl, das ausschließlich aus Kohlenstoffatomen und Wasserstoffatomen besteht, und
- mindestens ein funktionalisiertes synthetisches Wachs, das unter geradkettigen $C_{20-60}$-Fettalkoholen ausgewählt ist.

**2.** Zusammensetzung ohne Transfer nach Anspruch 1, **dadurch gekennzeichnet, dass** es sich bei dem flüchtigen Kohlenwasserstofföl um ein $C_{8-16}$-Isoparaffin oder um ein Gemisch von $C_{8-16}$-Isoparaffinen handelt.

**3.** Zusammensetzung ohne Transfer nach Anspruch 2, **dadurch gekennzeichnet, dass** das flüchtige Kohlenwasserstofföl Isododecan (2,2,4,4,6-Pentamethylheptan) ist.

**4.** Zusammensetzung ohne Transfer nach einem der vorhergehenden Ansprüche, **dadurch gekennzeichnet, dass** das flüchtige Kohlenwasserstofföl 5 bis 90 Gew.-%, vorzugsweise 5 bis 80 Gew.-% und besser noch 10 bis 60 Gew.-% der gesamten Zusammensetzung ausmacht.

**5.** Zusammensetzung ohne Transfer nach einem der vorhergehenden Ansprüche, **dadurch gekennzeichnet, dass** das funktionalisierte synthetische Wachs oder die funktionalisierten synthetischen Wachse 5 bis 30 Gew.-% und vorzugsweise 8 bis 20 Gew.-% der gesamten Zusammensetzung ausmachen.

**6.** Zusammensetzung ohne Transfer nach einem der vorhergehenden Ansprüche, **dadurch gekennzeichnet, dass** sie ferner ein Wachs oder mehrere Wachse tierischen, pflanzlichen oder synthetischen Ursprungs enthält, die von den funktionalisierten synthetischen Wachsen verschieden sind.

**7.** Zusammensetzung ohne Transfer nach einem der vorhergehenden Ansprüche, **dadurch gekennzeichnet, dass** sie ferner ein flüchtiges Lösemittel oder mehrere flüchtige Lösemittel enthält, die von den flüchtigen Kohlenwasserstoffölen verschieden sind.

**8.** Zusammensetzung ohne Transfer nach einem der vorhergehenden Ansprüche, **dadurch gekennzeichnet, dass** sie ferner ein Silicongummi, vorzugsweise in einem Mengenanteil von höchstens 2 Gew.-%, enthält.

**9.** Zusammensetzung ohne Transfer nach einem der vorhergehenden Ansprüche, **dadurch gekennzeichnet, dass** sie ferner mindestens ein nicht flüchtiges Öl pflanzlichen, tierischen, mineralischen oder synthetischen Ursprungs, vorzugsweise in einem Mengenanteil im Bereich von 5 bis 60 Gew.-% der gesamten Zusammensetzung, enthält.

**10.** Zusammensetzung ohne Transfer nach einem der vorhergehenden Ansprüche, **dadurch gekennzeichnet, dass** sie ferner einen oder mehrere kosmetische Zusatzstoffe oder Wirkstoffe enthält, die unter Sonnenschutzfiltern, Radikalfängern für freie Radikale, Hydratisierungsmitteln, Vitaminen, Proteinen, Ceramiden, Mitteln zum Regulieren des pH-Werts, Antioxidantien, Konservierungsstoffen, Füllstoffen, Pigmenten und Farbstoffen, Emollientien, Mitteln gegen Schaumbildung, Parfums, grenzflächenaktiven Stoffen und Weichmachern ausgewählt sind.

**11.** Zusammensetzung ohne Transfer nach einem der vorhergehenden Ansprüche, **dadurch gekennzeichnet, dass** sie in Form eines festen oder pastösen Schminkprodukts oder Pflegeprodukts vorliegt, das wasserfrei ist oder als Emulsion vorliegt.

**12.** Zusammensetzung ohne Transfer nach Anspruch 11, **dadurch gekennzeichnet, dass** es sich bei dem Schminkprodukt um einen Lippenstift, ein Make-up, einen Lidschatten, ein Wangenrouge oder um eine Mascara handelt.

**13.** Zusammensetzung zum Schminken ohne Transfer nach Anspruch 11, **dadurch gekennzeichnet, dass** das feste Schminkprodukt ein Stift für einen Lippenstift, ein Make-up oder ein gepresster Puder ist.

**14.** Verwendung der Kombination mindestens eines flüchtigen Kohlenwasserstofföls, das ausschließlich aus Kohlenstoffatomen und Wasserstoffatomen besteht, und mindestens eines funktionalisierten synthetischen Wachses, das unter geradkettigen $C_{20-60}$-Fettalkoholen ausgewählt ist, zur Herstellung eines kosmetischen Produkts zum Schminken oder zur Pflege ohne Transfer.

**15.** Verfahren zum Eindämmen des Transfers einer Zusammensetzung zur Pflege oder zum Schminken der Haut, der Lippen oder der Keratinfasern auf eine Oberfläche, mit der die Haut, die Lippen oder die Fasern in Kontakt gebracht werden, das darin besteht, in die Zusammensetzung die in den Ansprüchen 1 bis 5 definierte Kombination einzuarbeiten.

**Claims**

**1.** Transfer-resistant make-up or care composition, **characterized in that** it comprises, in a physiologically acceptable medium, the combination of

- at least one volatile hydrocarbon-based oil made up solely from carbon atoms and hydrogen atoms and
- at least one functionalized synthetic wax chosen from $C_{20-60}$ linear fatty alcohols.

2. Transfer-resistant composition according to Claim 1, **characterized in that** the volatile hydrocarbon-based oil is a $C_{8-16}$ isoparaffin or a mixture of $C_{8-16}$ isoparaffins.

3. Transfer-resistant composition according to Claim 2, **characterized in that** the volatile hydrocarbon-based oil is isododecane (2,2,4,4,6-pentamethylheptane).

4. Transfer-resistant composition according to any one of the preceding claims, **characterized in that** the volatile hydrocarbon-based oil represents from 5 to 90%, preferably from 5 to 80% and better still from 10 to 60% by weight, of the total composition.

5. Transfer-resistant composition according to any one of the preceding claims, **characterized in that** the functionalized synthetic wax(es) represent(s) from 5 to 30% and preferably from 8 to 20% by weight of the total composition.

6. Transfer-resistant composition according to any one of the preceding claims, **characterized in that** it also comprises one or more waxes of animal, plant or synthetic origin other than the functionalized synthetic waxes.

7. Transfer-resistant composition according to any one of the preceding claims, **characterized in that** it also comprises one or more volatile solvents other than volatile hydrocarbon-based oils.

8. Transfer-resistant composition according to any one of the preceding claims, **characterized in that** it also comprises a silicone gum, preferably in an amount of not more than 2% by weight.

9. Transfer-resistant composition according to any one of the preceding claims, **characterized in that** it also comprises at least one non-volatile oil of plant, animal, mineral or synthetic origin, preferably in an amount ranging from 5 to 60% by weight of the total composition.

10. Transfer-resistant composition according to any one of the preceding claims, **characterized in that** it also comprises one or more cosmetic adjuvants or active principles chosen from sunscreens, free-radical scavengers, hydrating agents, vitamins, proteins, ceramides, pH regulators, antioxidants, preserving agents, fillers, pigments, dyes, emollients, antifoaming agents, fragrances, surfactants and plasticizers.

11. Transfer-resistant composition according to any one of the preceding claims, **characterized in that** it is in the form of a solid or pasty make-up or care product, which is in anhydrous or emulsion form.

12. Transfer-resistant composition according to Claim 11, **characterized in that** the make-up product is a lipstick, a foundation, an eyeshadow, a face powder or a mascara.

13. Transfer-resistant make-up composition according to Claim 11, **characterized in that** the solid make-up product is a stick of lipstick, a foundation or a compact powder.

14. Use of the combination of at least one volatile hydrocarbon-based oil made up soley of carbon atoms and hydrogen atoms and at least one functionalized synthetic wax chosen from $C_{20-60}$ linear fatty alcohols. for the manufacture of a transfer-resistant make-up or care cosmetic product.

15. Process for limiting the transfer of a make-up or care composition from the skin, the lips or keratin fibres onto a surface with which the skin, the lips or the fibres are placed in contact, this process consisting in introducing the combination as defined in Claims 1 to 5 into the composition.